# EUROPEAN PATENT SPECIFICATION

(11) **EP 4 000 451 B1**
(45) Date of publication and mention of the grant of the patent: **24.07.2024**
(21) Application number: 20844439.8
(22) Date of filing: 05.06.2020
(51) Int. Cl.: A45D 44/22, A61N 1/32, A61N 1/04

(54) **COSMETIC MASK FOR EYES**
KOSMETISCHE AUGENMASKE
MASQUE COSMÉTIQUE POUR LES YEUX

(30) Priority: 19.07.2019 JP 2019133328
(43) Date of publication of application: 25.05.2022
(73) Proprietor: Ya-Man Ltd., Tokyo 135-0045 (JP)
(72) Inventor: YAMAZAKI, Iwao, Tokyo 135-0045 (JP)
(74) Representative: Kayser, Christoph
(86) International application number: PCT/JP2020/022217
(87) International publication number: WO 2021/014784

(56) References cited:
- WO-A1-2015/188811
- WO-A1-2019/093042
- FR-A1- 3 071 743
- JP-A- 2004 216 069
- JP-A- 2004 222 741
- JP-A- 2018 187 323
- JP-A- H03 264 078
- US-A1- 2002 161 416
- US-A1- 2007 276 451
- US-A1- 2018 161 579

## Description

### Technical Field

The present invention relates to a beauty mask for the skin around the eyes according to the preamble of claim 1.

### Background Art

WO 2015/188811 A1 discloses a beauty mask for the skin around the eyes fitted to the skin around the eyes. In this mask an electrode section is configured to exert an electrical stimulation on the temporal muscles. However, a stimulation on the orbicularis oculi muscles is not possible therewith.
Beside this a beauty mask is conventionally known which is fitted to the skin around the eyes of a user to perform various types of beauty care.

For example, patent literature 1 discloses an electrical stimulation mask in which a mask body section is made of silicone, in which opening sections are provided in the positions of eyes and in which a plurality of electrodes are arranged. Since the electrodes are arranged around the eyes, electric stimulation can be provided to the vicinity of eyes.

Patent literature 2 discloses a health appliance where a mask in which electrodes are installed in positions corresponding to neuromuscular stimulation points, which is formed with a soft membrane that is closely fitted onto the face, and where a pulse current is applied to achieve beauty effects and health effects provided by massage. The health appliance includes openings that correspond to the eyes, and electrodes are arranged around the eyes.

Patent literature 3 discloses a facial electrical stimulator in which a plurality of electrodes are arranged on a mask for the area around frontal eyes. It is disclosed that the electrodes are arranged on the upper section of an orbicularis oculi muscle and the lower section of the orbicularis oculi muscle.

Patent literature 4 discloses a facial instrument which includes a holding section that is fitted to the face of a user and electrodes that are arranged on positions along the direction of fibers of the orbicularis oculi muscle of the face when the holding section is fitted.

Patent literature 5 discloses a facial instrument which includes a holding section that is detachably fitted to the face of a user and an electrode section that abuts on the skin around the eyes. In the holding section, window sections in which openings are formed at the location of the eyes.

Patent literature 6 discloses a skin electrical stimulator in the shape of an eye mask, in which a plurality of electrodes are arranged so as to abut on the periphery of eyes. Holes are formed at the positions of the eyes.

Patent literature 7 discloses a beauty appliance for care of the skin around the eyes, in which a low frequency pulse current is supplied between a first skin electrode and a second skin electrode arranged on a mask body to apply a current acting on the first skin electrode that cares the skin around the eyes.

As disclosed in many patent literatures as mentioned above, configurations of beauty mask fitted to the skin around the eyes that exerts electrical beauty effects on muscles around the eyes are widely known.

However, a plurality of problems are left unsolved in the conventional configurations. Specifically, in the technique of patent literature 1, while a right side surface conductive section and a left side surface conductive section are provided, as a stimulator, which is not specifically disclosed, that applies electricity is externally connected to the electrodes using an output wire. Therefore, the stimulator needs to be provided separately and the wiring is inconvenient for handling. In addition, it is difficult to clean the electrodes which are in contact with the skin.

Patent literature 1 discloses a fitting band that is used by closely fitting the mask to the face in a stable manner. However, because of core material provided in the periphery of the mask, while it is possible to uniformly apply a force over the user's face, any effect of tightening the face conforming to convexo-concave on the face to remove slacks and the like is not provided.

In patent literature 3, while the electrodes are arranged to have a plurality of spots over the entire face, as in patent literature 1, it is inconvenient for handling as it is configured that electrical current applying an electrical stimulator is provided separately from the mask.

In patent literature 4, while electrode sections are arranged in a dotty pattern around the eyes, a current is likewise applied from an electrical pulse output means provided separately from the mask. It is provided with a plurality of electrode sections configured as to apply electric current from an electrical pulse output means provided separately from the mask. In patent literature 5, while electrode sections are arranged in a dotty pattern around the eyes, a current is likewise applied with a controller and an electrical code provided separately from the mask.

In patent literature 6, while a plurality of conductive sections are arranged in a dotty pattern, a current is likewise applied from a power supply control section provided separately from the mask.

In patent literature 7, while a first skin electrode is provided under the eyes and a second skin electrode is provided in an ear hook part, a current is likewise applied from a power supply source provided separately from the mask.

In patent literature 2, electrode sections are arranged to have a plurality of spots all over the mask, and an electrical pulse generator is installed at the spot corresponding to glabella on the mask. It is described that pulses generated from the electrical pulse generator are passed through several conductors to the electrodes.

When a plurality of electrodes are wired from the spot corresponding to glabella, due to the complicated structure because of the arrangement of the conductors, these may result in contact failure and poor maintainability. Disadvantageously, it is also difficult to clean the electrodes which are in contact with the skin.

Furthermore, while it is disclosed that the electrical pulse generator is driven with a battery, a specific configuration such as detachability of the battery is not disclosed.

The techniques of patent literatures 2 to 7 do not disclose that a mask including electrodes is closely fitted to the face, and an effect of tightening the face conforming to convexo-concave on user's face that removes slacks and the like is not provided. For example, patent literature 5, while a holding section which includes electrodes around the eyes with an elastic band section is fitted, the holding section is in the shape of a pair of goggles that cannot cover the facial line. patent literature 5 is configured to fit a holding section provided with electrodes around the eyes fitted with an elastic band section, it does not conform to a facial shape as the holding section has a shape of a pair of goggles.

### Citation List

### Patent Literature

Patent Literature 1: Japanese Patent No. 5650872
Patent Literature 2: Japanese Utility Model Registration No. 3137239
Patent Literature 3: Japanese Unexamined Patent Application Publication No. 2003-339884
Patent Literature 4: Japanese Unexamined Patent Application Publication No. 2004-222741
Patent Literature 5: Japanese Patent No. 4151418
Patent Literature 6: Japanese Unexamined Patent Application Publication No. 3-264078
Patent Literature 7: Japanese Patent No. 6043641

### Summary of Invention

### Technical Problem

The present invention is made to solve the problems in the conventional techniques described above, and an object thereof is to provide a beauty mask for the skin around the eyes, which achieves beauty effects such as electrical stimulation on the skin around the eyes, which effectively provides care for slacks by closely adhering the mask to accurate locations of the skin to provide stimulation, which is excellent in operability and maintainability including cleaning, recharging, as well as in beauty and design.

### Solution to Problem

In order to solve the problems described above, the present invention provides a beauty mask for the skin around the according to claim 1, ie. which is characterized in that the second electrode is provided in the stimulator and the stimulator storage section includes an extension region in which the second electrode section is stored.

### Summary of the disclosure

In a first embodiment, a beauty mask for the skin around the eyes fitted to the skin around the eyes of a user is provided, which includes: a right eye hole and a left eye hole that are openings corresponding to both eyes; a nose pad section that is formed at the center of a lower side of the beauty mask and that abuts on the nose root section of a user; a stimulator storage section that is formed on at least one of a right side of the right eye hole and a left side of the left eye hole; and a left or right fitting member that extends in at least one of leftward direction and rightward direction wound around the occiput to be fastened, and further includes a beauty mask body that conforms to the shape of the user's face due to elasticity and a stimulator that is stored in the stimulator storage section and that electrically simulates at least one of temporal muscle and an orbicularis oculi muscle.

In the second embodiment the stimulator storage section can be formed on the skin surface of a user to be concave, the stimulator can be detachably fitted into the stimulator storage section and the beauty mask for the skin around the eyes can include, on the skin side of the stimulator, a first electrode section that provides the electric stimulation.

In the third embodiment the beauty mask for the skin around the eyes may include, on an exterior surface of the stimulator, an operation section for operating the stimulator, and a concave bottom side of the stimulator storage section may be flexible such that the operation section can be operated from outside of the beauty mask body.

In the fourth embodiment the beauty mask for the skin around the eyes can include, over or under the right and left eye holes, a second electrode section that stimulates the orbicularis oculi muscle.

In the fifth embodiment the beauty mask for the skin around the eyes may include a second electrode section in the stimulator, and the stimulator storage section may include an extension region in which the second electrode section is stored.

In the sixth embodiment the first electrode section can have a first bending rigidity at least in the left-right direction of the area around eyes, and the second electrode section can have a second bending rigidity smaller than the first bending rigidity at least in the left-right direction of the area around the eye.

In the seventh embodiment the second electrode section may be provided detachably with respect to the first electrode section, and a detachable region for the second electrode section may be provided in the vicinity the skin around the eyes.

In the eighth embodiment the beauty mask body may be configured to provide a second electrode section, with the above stimulator equipped with an energizing terminal that supplies power to the second electrode section.

In the ninth embodiment the beauty mask for the skin around the eyes can include an upper fitting member that extends upward from the vicinity of the base section of the left and right fitting members and that are wound on the vertex or the occiput.

In the tenth embodiment currents of different frequencies may be applied from the first electrode section and the second electrode section so as to exert different types of electric stimulation to the orbicularis oculi muscle and the temporal muscle.

### Advantageous Effects

The beaut mask described herein achieves the following effects with the configurations described above.

Specifically, the beauty mask for the skin around the eyes can effectively provide care for slacks, as it can be adapted to the shape of the user's face due to elasticity. The distance between the right and left eye holes is adjustable to conform with the user's face, the beauty mask for the skin around the eyes can not only enhance the beauty effects but improve safety, as the stimulators provided on both sides can apply electric stimulation at an accurate spot by adjusting the position with the nose pad section abutting to the nose root.

Moreover, since the stimulator storage section to store the stimulator is provided in the beauty mask body, inconvenience of handling because the stimulator is provided separately in the prior arts is solved. Furthermore, the stimulator is formed to be concave on the skin side of the user and is detachably fitted into the mask, which improves the design property as well.

It is possible to enhance the adherability of the beauty mask for the skin around the eyes, as the stimulator is detachable and replaceable with a stimulator of an optimal shape that conforms to the user. Various beauty effects can be achieved as the stimulator by replacing with different stimulators having different beauty effects.

By providing a second electrode section over or under the right and left eye holes, the stimulator can stimulate the temporal muscles on both sides of the eyes, as well as orbicularis oculi muscles. Here, by providing a second electrode to the stimulator itself, wiring will not be required and multiple beauty effects will be very easily obtained.

In particular, it is possible to fit the electrodes that conforms to the facial line of a user, as a first electrode section has a first bending rigidity at least in the left-right direction at the vicinity of eyes, a second electrode section is configured with a second bending rigidity smaller than the first bending rigidity at least in the left-right direction at the vicinity of the eyes.

Moreover, it is possible to further improve the fitness by providing a movable region of a second electrode section at the vicinity of the eyes, as the second electrode section is provided detachably with respect to the first electrode section and a movable region of a second electrode in the vicinity of the skin around the eyes.

As it is provided with the stimulators on each side of eye holes, the energization terminal can directly supply power without wiring.

### Brief Description of Drawings

FIG. 1 is a perspective view of the beauty mask for the skin around the eyes according to the present invention;
FIG. 2 is a back view of the beauty mask for the skin around the eyes according to the present invention;
FIG. 3 is an enlarged view of the stimulator storage section according to the present invention; FIG. 4 is a perspective view of the stimulator according to the present invention;
FIG. 5 is an enlarged view of the operation section according to the present invention;
FIG. 6 is a cross-sectional view of the stimulator storage section according to the present invention; and
FIG. 7 is an illustrative view showing another example of the stimulator and the stimulator storage section.

### Description of Embodiments

An embodiment of the present invention will be described below based on examples shown in drawings. The embodiment of the present invention is not limited to the following description.

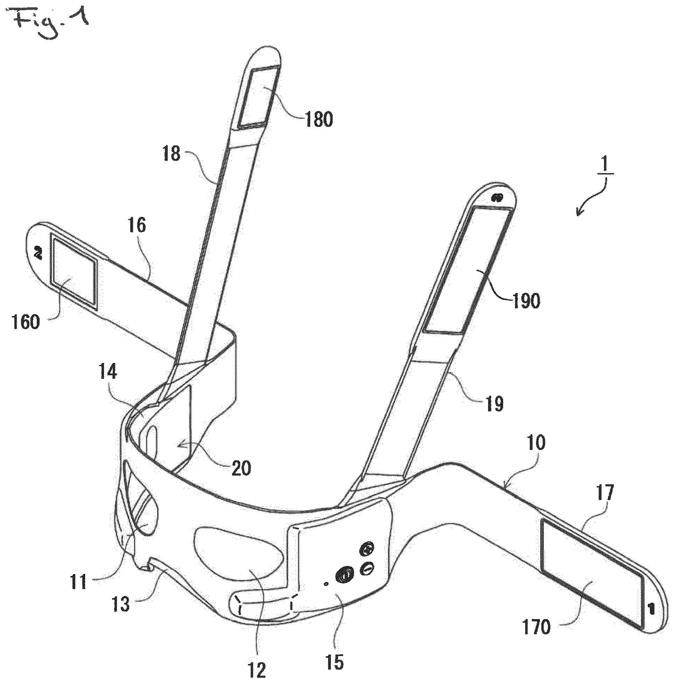

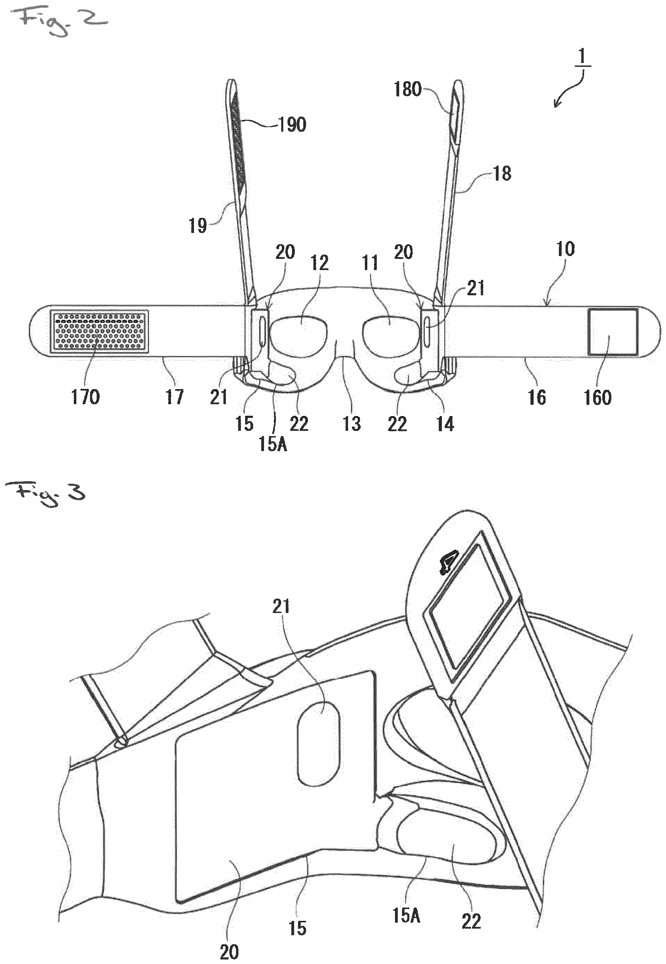

The beauty mask of the present invention is a beauty mask for the skin around the eyes, which is fitted to the skin around the eyes of a user. FIG. 1 shows a perspective view of the beauty mask for the skin around the eyes according to the present invention, and FIG. 2 is a back view thereof.

The beauty mask for the skin around the eyes (1) is formed with a beauty mask body (10) and stimulators (20), which electrically stimulate predetermined locations on the skin around the eyes.

On the beauty mask body (10), a right eye hole (11) and a left eye hole (12) which are opened in locations corresponding to eyes, a nose pad section (13) which is formed at the center of a lower side of the beauty mask body (10) and which abuts on the nose root section of a user and stimulator storage sections (14) and (15) which are formed at the right side of the right eye hole and at the left side of the left eye hole.

Left and right fitting members (16) (17) extend from the left and right sides of the beauty mask body (10) that are wound around the occiput to fasten the mask body (10) with hook-and-loop fasteners (160) (170) provided at the end sections thereof.

Since the beauty mask body (10) of the present invention is made of elastic material such as silicone or natural rubber, when the left and right fitting members (16) (17) are pulled in up-down directions, the right and the left eye hole sections can be expanded and contracted so that they fit to the position of the user's eyes due to elasticity.

In the meantime, according to the present invention, the line on the forehead between the central part of the nose, to which the nose pad is in close contact, and the upper side of the beauty mask body (10), is pulled from behind and brought into closer contact with the face, thereby making it adhere to the convexo-concave on the face such as the sides of the nose, as well as the recesses under and over the eyes.

In this respect, although the conventional beauty mask applied forces to the back of the face, it was configured to press the entire mask against the face of user at the base of the tensile belt, it failed to be appropriately applied over the convexo-concave around the eyes. Particularly for Japanese whose area around eyes is relatively flat, in an attempt to fit the electrodes, they could be excessively pressing against the protruding parts of cheek, eat into cheek, or be partly loose.

In the present invention, the beauty mask body (10) is adapted to convexo-concave to be able to effectively provide care for slacks, and the face suitably makes contact with the electrodes.

Since the beauty mask body (10) can be fixed to the occiput with an appropriate tensile force due to elasticity, it is unlikely to hurt the face hurt and to leave fitting mark that the user can comfortably use it.

In the present invention, the positions of the stimulators (20) can be adjusted simultaneously as the distance between the right eye hole (11) and the left eye hole (12) is adjusted. A configuration in which the area between the right eye hole (11) and the left eye hole (12) is expanded and contracted to be able to adjust the distance, or a configuration in which the vicinity of the right eye hole (11) and the left eye hole (12) are expanded and contracted to adjust the distance may be adopted. Naturally, a configuration in which the entire beauty mask body (10) including both parts is expanded and contracted may be adopted.

Here, it is preferable that the distance of the stimulator storage section (15) is 10 mm or greater from the right eye hole portion (11) and the left eye hole portion (12).

In the present example, while left and right fitting members (16) and (17) are extended to have substantially the same length on the left and right, a configuration with a fitting member provided on one of the left and right sides to be wound around the occiput, and in which its end section is then fastened to the beauty mask body, or a configuration with the lengths of the left and right fitting members differ from each other may be adopted.

The left and right fitting members may be detachable.

In this embodiment, the left and right fitting members (16) and (17) are further provided with the upper fitting members (18) and (19) extending upward from the vicinity of the base and being wound around the vertex or the occiput. The upper fitting section (18) and (19) is provided with hook-and loop fasteners (180) and (190) at the end section that can be fastened on vertex or the occiput.

By providing the upper fitting members (18) and (19) the beauty mask body (10) can be fitted stably, without shifting up and down, and by distributing the tensile force with the left and right fitting member, the burden can be reduced.

As shown in the figure, the upper fitting members (18) and (19) are provided so as to be inclined rearward within a range of 5 to 20 degrees, not directly above the vicinity of the bases of the left and right fitting members (16) and (17). According to this configuration, it is possible to further improve the usability by fixing it at a place slightly closer to the occiput than the vertex with a relatively little burden.

In the present invention, the beauty mask main body (10) made of elastic material as described above that can achieve lift-up effect by physically pulling up the skin around eyes and slack removing effect by applying appropriate tensile force from the left and right fitting members, as well as from the upper fitting member. Therefore, it is possible to provide a beauty mask for the skin around eyes that has a beauty effect by merely wearing the mask, which function is not limited to the electrical stimulation by the stimulator described below.

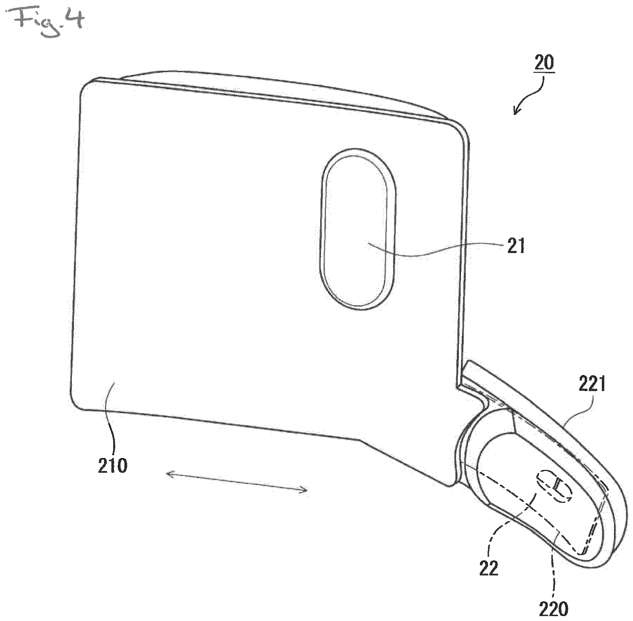

FIG. 3 is an enlarged view of the stimulator storage section according to the present invention, that shows an enlarged view of the fitted stimulator (20) and the stimulator storage section (15) on the left of the left eye seen from the back. Moreover, FIG.4 is a perspective view of the stimulator (20) according to the invention, that shows its back side view, i.e., the section facing the skin surface of a user.

The beauty mask for the skin around the eyes (1) is used in a state where the stimulator (20) is fitted into the stimulator storage section (15), and the stimulator (20) includes a first electrode section (21) acting on the temporal muscle and a second electrode section (22) acting on the orbicularis oculi muscle under the eyes.
As the stimulator (20) of the present invention has a bisymmetrical shape, while FIG. 4 and the following description details the left stimulator, the configuration for the right simulator is also the same.

The stimulator (20), which is rechargeable as it is embedded with a rechargeable battery, exerts an electrical muscle stimulation referred as EMS (Electrical Muscle Stimulation) that applies electric currents at predetermined frequencies using the stored electric power. It is widely known that when the EMS, electric stimulation, is applied to the skin at frequencies of several Hz to several kHz, it generates the muscle contraction that performs muscle exercise and relieves tension.

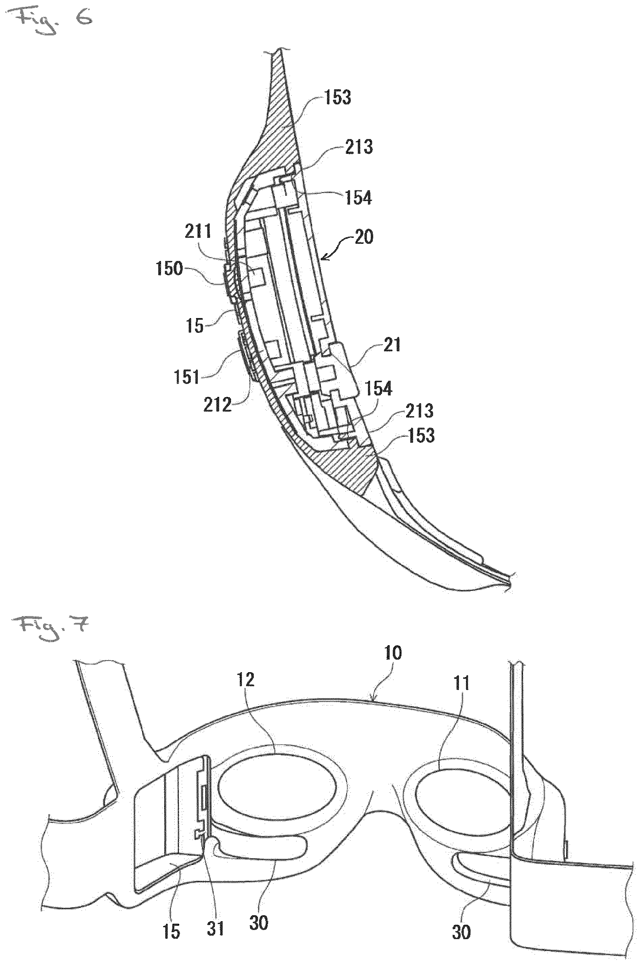

The stimulator (20) is provided with a control circuit for generating EMS and an operation switch (121,212 in FIG. 6) for operating the control circuit on the front side, that applies the current generated by the control circuit through the first electrode section (21) to the skin surface. It can be configured that, while the beauty mask body (10) is made of a conductive material that is energized by connecting it with a control circuit, it can be provided in parallel with the first electrode section (21) on the stimulator (20). It may be energized with the second electrode section (22).

The first electrode section (21) has a vertically long oval shape, and generates a low frequency current of about 100 Hz along the temporal muscle. According to the inventors, the tension of the temporal muscle is effectively relieved at frequencies of about 50 Hz to 200 Hz.

On the other hand, the second electrode section (22) is formed extending the stimulator (20) under the right eye hole (11) and the left eye hole (12). In order to fit the second electrode section (22), the stimulator storage section (15) includes an extension region (15A) in which the second electrode section (22) is stored.

The second electrode section (22) is not necessarily fitted into the extension region (15A), and the second electrode section (22) may be pressed against the inner surface simply by the beauty mask body (10).

The second electrode section acts on the orbicularis oculi muscle, and applies a low frequency current of about 25 Hz generated from the control circuit. Inventors suggest that it be effective to stimulate and train the orbicularis oculi muscle at frequencies of about several Hz to 50 Hz.

In the present invention, the beauty effects on the skin around the eyes, such as slacks can be significantly improved, as the lifting-up effect performed by the beauty mask body is combined with an effect of releasing the temporal muscle with the first electrode section (21) and an effect of training the temporal muscle with the second electrode section (22). The present invention is significantly effective, as no product equipped with the effects based on the combination described above has been provided conventionally.

In the present invention, it is more preferable to differ the bending rigidity of the second electrode section (22) from that of the vicinity of the first electrode section (21).

Specifically, as shown in FIG. 4, the configuration of the second electrode section (22) that houses an internally flexible thin plate (220), which is covered with a covering member (221) made of conductive rubber.

The stimulator body (210) provided with the first electrode section (21) is made of highly rigid resin and is hard to be bent in the left-right direction (left-right arrows as shown in the figure) of the area around eyes, and the second electrode section (22) can be easily deformed due to elasticity of the thin plate or conductive rubber.

Hence, the second electrode section (22) is adapted to a shape extending from the temporal region to the area around the eyes which differs depending on user, and the electrode appropriately abuts on the skin surface and reduces the load caused when wearing it.

The second electrode section (22) may be provided detachably with respect to the first electrode section (21). For example, a configuration may be adopted in which the first electrode section (21) and the second electrode section (22) are coupled together with a resin material such as rubber that can be freely moved or a detachable member such as a hinge or a ball joint, and the second electrode section (22) may include a detachable region on the skin around the eyes.

The second electrode section (22) may be detachably provided to the first electrode section (21). For example, a configuration may consist of the first electrode section (21) and the second electrode section (22) that are coupled together with resin material made of rubber and the like, that can be freely moved, or a detachable member such as hinge or ball joint, or it can be provided with the second electrode section (22) that has a detachable region on the area around the eyes.

With this configuration, it is possible to improve the effect of removing slacks by improving fitness of the second electrode section (22).
Detachable region can be configured to be detachable in left-right directions, as well as in up-down directions, and be freely curved in response to the skin surface owing to the flexibility of resin material and the ball joint.

The detachable section as mentioned above may be configured to exert an acting force against the skin surface of the user owing to elasticity of movable section of the first electrode section (21), the second electrode section (22) or the elasticity of each electrode section itself.
This configuration contributes to the beauty effects by pressing these sections with an appropriate force depending on the parts of the skin.

Since the stimulator (20) of the present invention is detachable, a plurality of types of stimulators which thickness and flexibility are adjusted according to the users may be provided. For example, by providing standardized facial shape stimulators (20) that are adapted to each racial property, each user can select one of the stimulators (20) optimal for oneself.

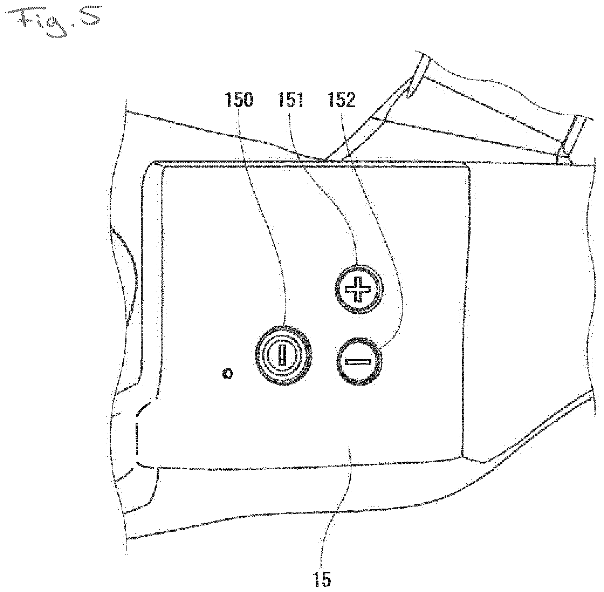

FIG. 5 is an enlarged view of an operation section viewed from the front side of the beauty mask body (10). In the present invention, the operation section includes a mode switch (150) for switching power supply and modes, a plus switch (151) and a minus switch (152) for adjusting the level and the like. Naturally, the type of switch to be provided can be optionally changed

In this configuration, the operation switches (211 and 212 in FIG. 6) provided on the exterior surface of the stimulator (20) can be operated from outside of the stimulator storage section (15) due to flexibility of the material. Specifically, operation switches corresponding to the stimulator (20) are arranged on the back side of the switches (150), (151) and (152).

According to this configuration, two major effects are provided. Firstly, if the fitting section of the stimulator is opened, the beauty mask for the skin around the eyes will lack durability, as it is relatively thin that consists of the nose pad section (13) at the lower side and the area above both eye holes at the upper side. The configuration of the present invention is suitable to allow the direct effect of the stimulator provided on the skin surface side that has no opening while it can reliably hold the stimulator.

Secondly, as water does not directly come into contact with the operation switches of the stimulator, stain and failure can be prevented, even when the beauty mask for the skin around the eyes is handled with wet hands because it is often used in a wet environment along with skin lotion and the like, for instance. In this respect, the configuration of the present invention is preferable because the beauty mask for the skin around the eyes can be used without direct touch to the stimulator.

FIG. 6 shows a cross-sectional view of the stimulator storage section (15) of the present invention when viewed from above. In the present invention, the stimulator storage section (15) is formed to be concave so as to be fitted into the external shape of the stimulator (20), and it can hold the stimulator (20) because a concave contour section (153) made thick. Further, a protruded section (154) is provided inside of the concave contour section (153), and is engaged with a flange section (213) of the stimulator (20) to align the surface of the stimulator storage section (15) and the surface of the stimulator (20) so as to form a continuous flat surface. Hence, it is possible to avoid an issue, for example, that the stimulator (20) is excessively buried into to prevent the first electrode section (21) from not appropriately abutting on the skin surface.

With the configuration of the stimulator storage section (15) and the stimulator (20) according to the present invention, as the stimulator is detachable, it can be easily cleaned and recharged. It is also easy to configure the beauty mask body (10) washable with water.

Furthermore, various devices offering other beauty effects like microcurrent for passing small current, iontophoresis and ultrasonic stimulation can be fitted as the stimulator (20) is made replaceable.

In the present example, while the second electrode sections (22) are provided under both eye holes, they may be shifted symmetrically in up-down directions to be provided over the eye holes. They may be provided on both upper and lower sides.

While the second electrode section indicates a mono-polar configuration, it may be a bi-polar configuration to perform energization therebetween. In this case, it can provide intensive treatment to the skin around the eyes.

In order to enhance blood circulation improvement under the skin around the eyes and a relaxation effect, heaters may be provided around the right eye hole (11) and the left eye hole (12). For example, an arc-shaped heater provided under each of the holes (11) and (12) is able to maintain the temperature at about 40 degrees Celsius with the stimulator (20) that supplies power through an energization terminal.

In particular, an effect of preventing dark circles under the eyes can be achieved, by training the orbicularis oculi muscle and blood circulation promotion using the heater of the present invention.

FIG. 7 is an illustrative view showing another example of a stimulator and a stimulator storage section. In the example of FIG. 7, while the second electrode section is not provided in the stimulator (20), second electrode regions (30), which are integrally made of a conductive material are provided under the right eye hole (11) and the left eye hole (12) of the beauty mask body (10). In the second electrode region (30), an energization terminal (31) for performing energization from the stimulator is provided.

In this configuration, effective beauty care can likewise be performed on the temporal muscle and the orbicularis oculi muscle.

### Reference Signs List

- 1: beauty mask for the skin around the eyes
- 10: beauty mask body
- 11: right eye hole
- 12: left eye hole
- 13: nose pad section
- 14, 15: stimulator storage sections
- 15A: extension region
- 150: mode switch
- 151: switch
- 152: switch
- 153: contour section
- 154: protruded section
- 16, 17: left and right fitting members
- 18, 19: upper fitting members
- 160, 170: hook-and-loop fasteners
- 20: stimulator
- 21: first electrode section
- 210: stimulator body
- 211, 212: operation switches
- 213: flange section
- 22: second electrode section
- 220: thin plate
- 221: covering member
- 30: second electrode region
- 31: energization terminal

## Claims

1. A beauty mask (1) for the skin around the eyes of a user, configured to be fitted to the skin around the eyes of the user, comprising:
a right eye hole (11) and a left eye hole (12) each corresponding to one of the eyes;
a nose pad section (13) that is formed in a center section of the lower side of the beauty mask and configured to abut on the nose root section of the user; a stimulator storage section (14; 15) that is formed on at least one of a right side of the right eye hole (11) and a left side of the left eye hole (12); and
wherein the beauty mask (1) for the skin around the eyes is made of elastic material so as to conform to the shape of the user's face, and
includes left and right fitting members (16; 17) extending in a leftward and rightward direction respectively, the left and right fitting members being configured to be wound around the occiput of the user and to be fastend; and
a stimulator (20) having a first electrode section (21) stored in the stimulator storage section (14; 15) and configured to exert an electrical stimulation on the user's temporal muscles, and a second electrode section (22) configured to stimulate the user's orbicularis oculi muscles over or under the right and left eye holes (11; 12),
wherein the stimulator (20) is detachably fitted into the stimulator storage section (14; 15);
**characterized in, that**
the second electrode section (22) is provided in the stimulator (20) and the stimulator storage section (14; 15) includes an extension region (15A) in which the second electrode section (22) is stored.

2. The beauty mask (1) for the skin around the eyes according to claim 1,
wherein the stimulator storage section (14; 15) is formed to be concave on the side intended to face the user's skin.

3. The beauty mask (1) for the skin around the eyes according to claim 2, wherein
an operation section for operating the stimulator (20) is provided on an exterior surface of the stimulator (20), and
a corresponding external side of the stimulator storage section (14; 15) is flexible so that the operation section can be operated from outside of the beauty mask.

4. The beauty mask (1) for the skin around the eyes according to one of claims 1 to 3,
wherein the first electrode section (21) has a first bending rigidity at least in the left -right direction and
the second electrode section (22) has a second bending rigidity that is smaller than the first bending rigidity at least in the left -right direction.

5. The beauty mask (1) for the skin around the eyes according to claim 4,
wherein the second electrode section (22) is provided detachably attached to the first electrode section (21).

6. The beauty mask (1) for the skin around the eyes according to one of claims 1 to 3,
wherein the stimulator (20) is provided with an energizing terminal (31) that energizes the second electrode section (22),
and the stimulator (20) is configurated to supply power to the second electrode section (22).

7. The beauty mask (1) for the skin around the eyes according to any of claims 1 to 6, further comprising upper fitting members (18; 19) extending upward from the vicinity of a base section of the left and right fitting members (16; 17) and that are configurated to be wound around the vertex or the occiput of the user.

8. The beauty mask (1) for the skin around the eyes according to claim 1 to 3, the stimulator is configured to apply currents of different frequencies to the first electrode section (21) and the second electrode section (22) to provide different types of electric stimulation to the orbicularis oculi muscle and the temporal muscle.

## Patentansprüche

1. Schönheitsmaske (1) für die Haut rund um die Augen eines Benutzers, die dazu ausgestaltet ist, an die Haut rund um die Augen des Benutzers angelegt zu werden, umfassend:
ein rechtes Augenloch (11) und ein linkes Augenloch (12), die jeweils einem der Augen entsprechen;
einen Nasenkissenabschnitt (13), der in einem mittigen Abschnitt der Unterseite der Schönheitsmaske ausgebildet und dazu ausgestaltet ist, auf dem Nasenwurzelabschnitt des Benutzers aufzuliegen;
einen Stimulatoraufbewahrungsabschnitt (14; 15), der auf wenigstens einer aus einer rechten Seite des rechten Augenlochs (11) und einer linken Seite des linken Augenlochs (12) ausgebildet ist; und
wobei die Schönheitsmaske (1) für die Haut rund um die Augen aus elastischem Material hergestellt ist, um sich an die Form des Gesichts des Benutzers anzupassen, und
ein linkes und ein rechtes Anlegeelement (16; 17) umfasst, die sich entsprechend in einer nach links weisenden und nach rechts weisenden Richtung erstrecken, wobei das linke und rechte Anlegeelement dazu ausgestaltet sind, um den Hinterkopf des Benutzers gewickelt und befestigt zu werden; und
einen Stimulator (20) mit einem ersten Elektrodenabschnitt (21), der in dem Stimulatoraufbewahrungsabschnitt (14; 15) aufbewahrt wird und dazu ausgestaltet ist, eine elektrische Stimulation auf die Schläfenmuskeln des Benutzers auszuüben, und einem zweiten Elektrodenabschnitt (22), der dazu ausgestaltet ist, die Augenringmuskeln des Benutzers zu stimulieren, über oder unter dem rechten und linken Augenloch (11; 12),
wobei der Stimulator (20) lösbar in den Stimulatoraufbewahrungsabschnitt (14; 15) eingelegt ist;
**dadurch gekennzeichnet, dass**
der zweite Elektrodenabschnitt (22) in dem Stimulator (20) vorgesehen ist und der Stimulatoraufbewahrungsabschnitt (14; 15) einen Verlängerungsbereich (15A) umfasst, in dem der zweite Elektrodenabschnitt (22) aufbewahrt wird.

2. Schönheitsmaske (1) für die Haut rund um die Augen nach Anspruch 1,
wobei der Stimulatoraufbewahrungsabschnitt (14; 15) so ausgebildet ist, dass er auf der Seite, die der Haut des Benutzers zugewandt sein soll, konkav ist.

3. Schönheitsmaske (1) für die Haut rund um die Augen nach Anspruch 2,
wobei ein Bedienabschnitt zum Bedienen des Stimulators (20) auf einer Außenfläche des Stimulators (20) vorgesehen ist und
eine entsprechende Außenseite des Stimulatoraufbewahrungsabschnitts (14; 15) flexibel ist, sodass der Bedienabschnitt von außerhalb der Schönheitsmaske bedient werden kann.

4. Schönheitsmaske (1) für die Haut rund um die Augen nach einem der Ansprüche 1 bis 3,
wobei der erste Elektrodenabschnitt (21) eine erste Biegesteifigkeit wenigstens in der Links-rechts-Richtung aufweist und
der zweite Elektrodenabschnitt (22) eine zweite Biegesteifigkeit, die geringer als die erste Biegesteifigkeit ist, wenigstens in der Links-rechts-Richtung aufweist.

5. Schönheitsmaske (1) für die Haut rund um die Augen nach Anspruch 4,
wobei der zweite Elektrodenabschnitt (22) lösbar an dem ersten Elektrodenabschnitt (21) angebracht vorgesehen ist.

6. Schönheitsmaske (1) für die Haut rund um die Augen nach einem der Ansprüche 1 bis 3,
wobei der Stimulator (20) mit einem Energieversorgungsanschluss (31) versehen ist, der den zweiten Elektrodenabschnitt (22) mit Energie versorgt, und der Stimulator (20) dazu ausgestaltet ist, dem zweiten Elektrodenabschnitt (22) Leistung zuzuführen.

7. Schönheitsmaske (1) für die Haut rund um die Augen nach einem der Ansprüche 1 bis 6,
die ferner obere Anlegeelemente (18; 19) umfasst, die sich von der Nähe eines Basisabschnitts des linken und rechten Anlegeelements (16; 17) aus erstrecken und die dazu ausgestaltet sind, um den Oberkopf oder den Hinterkopf des Benutzers gewickelt zu werden.

8. Schönheitsmaske (1) für die Haut rund um die Augen nach Anspruch 1 bis 3,
wobei der Stimulator dazu ausgestaltet ist, Ströme mit verschiedenen Frequenzen an den ersten Elektrodenabschnitt (21) und den zweiten Elektrodenabschnitt (22) anzulegen, um verschiedene Arten elektrischer Stimulation für den Augenringmuskel und den Schläfenmuskel bereitzustellen.

## Revendications

1. Masque de beauté (1) destiné pour la peau autour des yeux d'un utilisateur, configuré pour être ajusté sur la peau autour des yeux de l'utilisateur, comprenant
un trou pour l'œil droit (11) et un trou pour l'œil gauche (12) correspondant chacun à l'un des yeux ;
une section de coussinet nasal (13) qui est formée dans une section centrale du côté inférieur du masque de beauté et configurée pour venir s'appuyer contre la section de la racine du nez de l'utilisateur ;
une section de stockage du stimulateur (14 ; 15) qui est formée sur au moins l'un des côtés droit du trou pour l'œil droit (11) et gauche du trou pour l'œil gauche (12) ; et
dans lequel le masque de beauté (1) destiné pour la peau autour des yeux est constitué d'un matériau élastique de manière à s'adapter à la forme du visage de l'utilisateur, et
comprend des éléments d'ajustement gauche et droit (16 ; 17) s'étendant respectivement dans une direction vers la gauche et vers la droite, les éléments d'ajustement gauche et droit étant configurés pour être enroulés autour de l'occiput de l'utilisateur et pour être attachés ; et
un stimulateur (20) comportant une première section d'électrode (21) stockée dans la section de stockage de stimulateur (14 ; 15) et configurée pour exercer une stimulation électrique sur les muscles temporaux de l'utilisateur, et une seconde section d'électrode (22) configurée pour stimuler les muscles orbicularis oculi de l'utilisateur situés au-dessus ou en dessous des orifices oculaires droit et gauche (11 ; 12),
dans lequel le stimulateur (20) est installé de manière amovible dans la section de stockage du stimulateur (14 ; 15) ;
**caractérisé en ce que**
la seconde section d'électrode (22) est prévue dans le stimulateur (20) et la section de stockage du stimulateur (14 ; 15) comprend une région d'extension (15A) dans laquelle la seconde section d'électrode (22) est stockée.

2. Masque de beauté (1) destiné pour la peau autour des yeux selon la revendication 1,
dans lequel la section de stockage du stimulateur (14 ; 15) est formée pour être concave sur le côté destiné à faire face à la peau de l'utilisateur.

3. Masque de beauté (1) destiné pour la peau autour des yeux selon la revendication 2,
dans lequel une section de fonctionnement pour faire fonctionner le stimulateur (20) est prévue sur une surface extérieure du stimulateur (20), et
un côté extérieur correspondant de la section de stockage du stimulateur (14 ; 15) est flexible de sorte que la section de fonctionnement puisse être actionnée depuis l'extérieur du masque de beauté.

4. Masque de beauté (1) destiné pour la peau autour des yeux selon l'une quelconque des revendications 1 à 3,
dans lequel la première section d'électrode (21) présente une première rigidité de flexion au moins dans la direction gauche-droite et
la seconde section d'électrode (22) présente une seconde rigidité de flexion qui est inférieure à la première rigidité de flexion au moins dans la direction gauche-droite.

5. Masque de beauté (1) destiné pour la peau autour des yeux selon la revendication 4,
dans lequel la seconde section d'électrode (22) est fixée de manière amovible à la première section d'électrode (21).

6. Masque de beauté (1) destiné pour la peau autour des yeux selon l'une quelconque des revendications 1 à 3,
dans lequel le stimulateur (20) est pourvu d'une borne d'alimentation (31) qui alimente la seconde section d'électrode (22), et le stimulateur (20) est configuré pour fournir de l'énergie à la seconde section d'électrode (22).

7. Masque de beauté (1) destiné pour la peau autour des yeux selon l'une quelconque des revendications 1 à 6,
comprenant en outre des éléments d'ajustement supérieurs (18 ; 19) s'étendant vers le haut à proximité d'une section de base des éléments d'ajustement gauche et droit (16 ; 17) et qui sont configurés pour être enroulés autour du vertex ou de l'occiput de l'utilisateur.

8. Masque de beauté (1) destiné pour la peau autour des yeux selon l'une quelconque des revendications 1 à 3,
dans lequel le stimulateur est configuré pour appliquer des courants de différentes fréquences à la première section d'électrode (21) et à la seconde section d'électrode (22) afin de fournir différents types de stimulation électrique au muscle orbicularis oculi et au muscle temporal.
